# EUROPEAN PATENT APPLICATION

(11) **EP 2 153 821 A1**
(43) Date of publication of application: **17.02.2010**
(21) Application number: 08305455.1
(22) Date of filing: 06.08.2008
(51) Int. Cl.: A61K 9/14, A61K 31/4745

(54) **Oral formulations of camptothecin derivatives**

(71) Applicant: BioAlliance Pharma, 75015 Paris (FR)
(72) Inventor: Bonnafous, David, 94500 CHAMPIGNY SUR MARNE (FR); Cave, Guy, 94240 L'HAY LES ROSES (FR); Dembri, Assia, 75006 PARIS (FR); Lebel-Binay, Sophie, 94800 VILLEJUIF (FR); Ponchel, Gilles, 75012 PARIS (FR); Soma, Emilienne, 75013 PARIS (FR)
(74) Representative: Blot, Philippe Robert Emile

(57) **Abstract**

The present invention is directed to new oral formulations of camptothecin derivatives, their process of preparation as well as their therapeutic uses.

More specifically, said invention is related to nanoparticles comprising at least one camptothecin derivative as an active ingredient, at least one polymer and at least one cyclic oligosaccharide capable of complexing said camptothecin derivative, said nanoparticles being for therapeutic oral administration.

## Description

### FIELD OF THE INVENTION

The present invention is directed to new oral formulations of camptothecin derivatives, their process of preparation as well as their therapeutic uses.

### BACKGROUND OF THE INVENTION

Cancer is characterized by uncontrolled growth of cells coupled with malignant behavior: invasion and metastasis. It is a major cause of mortality in most industrialized countries. Different ways of cancer treatment can be used: chemotherapy, radiotherapy, surgery, immunotherapy and hormonotherapy.

Chemotherapy can be defined as the use of cytotoxic drugs (also named "chemotherapeutic agents"), to treat cancer. Broadly, most chemotherapeutic agents work by impairing mitosis (cell division), effectively targeting fast-dividing cells. As these drugs cause damage to cells they are termed "cytotoxic".

Chemotherapeutic treatment can be physically exhausting for the patient. Current chemotherapeutic techniques have a range of side effects mainly affecting the fast-dividing cells of the body. Important common side effects include (dependent on the chemotherapeutic agent): nausea and/or vomiting, diarrhea or constipation, anemia, malnutrition, hair loss, memory loss, depression of the immune system, hence (potentially lethal) infections and sepsis, hemorrhage, secondary neoplasms, cardiotoxicity, hepatotoxicity, nephrotoxicity, neurotoxicity.

Camptothecin (CPT) is a cytotoxic quinoline alkaloid isolated from Camptotheca acuminata (Camptotheca, Happy tree), a tree native in China and Tibet. It inhibits the DNA enzyme topoisomerase I. Topoisomerase I, an intranuclear enzyme that noncovalently binds to torsionally strained, supercoiled, double-stranded DNA, creates a transient single-strand break (named "cleavable complex") in the DNA molecule. This allows for the passage of an intact complementary DNA strand during replication, transcription, recombination, and other DNA functions. The enzyme-bridged DNA breaks, also known as cleavable complexes, are then resealed by the topoisomerase I enzyme. Dissociation of the enzyme restores an intact, newly relaxed DNA double helix.

CPT stabilize the cleavable complex between the topoisomerase I molecule and the free 3'-phosphate of the DNA. The resulting enzyme-linked DNA breaks induce cytotoxicity. In the past, CPT has often been referred to as topoisomerase I inhibitor. However, it is not classic enzyme inhibitor since rather than directly altering the function of topoisomerase I, it converts this normal endogenous protein into a cellular toxin. Therefore, CPT is often preferentially referred to as topoisomerase I poison (Bomgaars and al. (2001) Oncologist 6(6): 506-16).

CPT showed anticancer activity in preliminary clinical trials and two CPT analogs have been approved and are used in cancer chemotherapy: (i) irinotecan (CPT - 11), marketed as Campto or Camptosar by UpJohn (now Pfizer) and (ii) topotecan, marketed as Hymcamptamin, Hycamptin, or Thycantin, by Smith Kline & Beecham (now GSK) (Ulukan, and al. (2002) Drugs 62 (2): 2039-2057). Other CPT analogs include hexatecan, silatecan, lutortecan, karenitecin (BNP1350), gimatecan (ST1481), belotecan (CKD602) or their pharmaceutically acceptable salts. However CPT analogs present significant drawbacks as low solubility and adverse effect.

Irinotecan ("IRN" or "CPT-11" or "Irinotecan hydrochloride") is a semisynthetic analogue of camptothecin and a well-known anticancer chemotherapeutic agent with a main indication in metastatic colorectal cancer. It is also studied for the treatment of lung cancers, stomach cancer, pancreas cancer, non-Hodgkin lymphoma, uterine cervix cancer, head and neck cancers, brain cancer and ovary cancer.

Irinotecan is a prodrug that is converted by carboxylesterase in the liver, intestinal tract, and some tumors to its active metabolite, SN-38 (7-ethyl10-hydroxycamptothecin). SN-38 is 100- to 1,000-fold more potent than irinotecan. SN-38 is then inactivated by glucuronidation by uridine diphosphate glucuronosyltransferases (UGT), to form SN-38 glucuronide (SN-38G or 7-ethyl-10-[3,4,5-trihydroxy-pyran-2-carboxylic acid]camptothecin) (Kawato et al., 1991 Cancer Chemother Pharmacol 28(3): 192-8; Takasuna et al., Cancer Res (1996) 56(16): 3752-7; Slatter et al., 1997 Metab Dispos 25(10): 1157-64)).

A drawback of irinotecan is severe adverse effects and notably severe diarrhea and extreme suppression of the immune system. Irinotecan-associated diarrhea may be clinically significant, sometimes leading to severe dehydration requiring hospitalization or intensive care unit admission. Irinotecan-associated suppression of the immune system leads to dramatically lowered white blood cell counts in the blood, in particular the neutrophils. While the bone marrow, where neutrophils are made, cranks up production to compensate, the patient may experience a period of neutropenia, that is, a clinical lack of neutrophils in the blood.

The efficacy of Irinotecan is known to be dose-dependent and has also been shown to be schedule-dependent, with prolonged low-dose administration being more effective and less toxic than short duration high-dose schedules (Houghton, P. J. et. al. "Efficacy of Topoisomerase I Inhibitors Topotecan and Irinotecan administered at Low Dose Levels in Protracted Schedules to Mice Bearing Xenografts of Human Tumors" Cancer Chemother. Pharmacol. (1995), 36, 393-403; Thompson, J. et. al. "Efficacy of Systemic Administration of Irinotecan Against Neuroblastoma Xenografts" Clin. Cancer Res. (1997), 3, 423-432; Furman WL and al, "Direct translation of a protracted irinotecan schedule from a xenograft model to a phase I trial in children" Journal of clinical oncology (1999), 17, 1815-1824).

An efficient approach to prolong exposure is to use the oral route. Besides several advantages of oral administration in general (e.g., patient's preference, convenience, reduced hospital stay, and reduced cytotoxic exposure risk for health care workers), more specific advantages can be distinguished. Indeed, compared with i.v. administration, the metabolic ratio of total SN-38 to total irinotecan after oral administration is higher (Gupta E and al., Pharmacokinetic and pharmacodynamic evaluation of the topoisomerase inhibitor irinotecan in cancer patients. J Clin Oncol 1997;15:1502-10).

Thus the need has arisen to develop oral formulations of camptothecin derivatives that would improve efficacity, bioavailability and ameliorate side effects.

It is therefore desirable to provide new formulations of Irinotecan allowing oral administration.

WO 99/43359 discloses nanoparticles comprising at least (i) one polymer, (ii) one cyclic oligosaccharide and (iii) one active ingredient. It has been described that these nanoparticles allow sustained (controlled) release of drug (Maincent P and al "Preparation and in vivo studies of a new drug delivery system. Nanoparticles of alkylcyanoacrylate", Appl Biochem Biotechnol. 1984;10:263-5) and have bioadhesion properties (Ponchel G and al. "Specific and non-specific bioadhesive particulate systems for oral delivery to the gastrointestinal tract", Adv Drug Deliv Rev. 1998 Dec 1;34(2-3):191-219). Said Nanoparticles are hereinafter referred to as "Sustained Released Nanoparticles" ("SRN").

The inventors have now surprisingly found that SRN containing camptothecin derivatives ("camptothecin-SRN") are suitable for oral administration of camptothecin derivatives. They have surprisingly shown that oral administration of camptothecin-SRN improve efficacy of camptothecin derivatives, reduce their side effects and increase their bioavailability. The SRN are thus highly advantageous in connection with camptothecin derivatives such as, but not only, irinotecan (irinotecan-SRN),

Indeed, in a non obviously way, camptothecin-SRN allow oral administration and sustained release of active ingredient, which help to improve its bioavailability and reduce its side effects. The bioadhesion of SRN on intestinal mucous membrane increases the residence time and thus reinforce the sustained release of the active ingredient.

Moreover, improving irinotecan residence time in the gastro-intestinal tract allows a higher conversion of irinotecan into its active metabolite SN38. In particular, it was found that the IRN-SRN were able to efficiently protect the active agent from the pH-dependent degradation such as in the gastro-intestinal environment. As a result, the Irinotecan's lactone form is protected from conversion to its inactive form (carboxylate) which would normally occur with oral administration, as IRN and SN38 are instable in the intestinal pH (pH 5,5 to 7).

### SUMMARY OF THE INVENTION

The present invention thus concerns nanoparticles ("SRN") comprising at least one camptothecin derivative as an active ingredient, at least one polymer and at least one cyclic oligosaccharide capable of complexing said camptothecin derivatives ("camptothecin-SRN"), for therapeutically oral administration of said camptothecin derivatives.

The present invention also concerns said camptothecin-SRN for the treatment and/or the prevention of cancer, said treatment and/or prevention comprising the oral administration of said camptothecin-SRN.

Preferably, said polymer is chosen from the poly(alkylcyanoacrylate) in which the alkyl group, linear or branched, comprises one to twelve carbon atoms.

Preferably, said polymer is the poly(isohexylcyanoacrylate). This polymer may be obtained from polymerisation of Monorex® (Bioalliance Pharma).

Preferably, said cyclic oligosaccharide is a neutral or charged cyclodextrin, native, branched or polymerised or chemically modified. It is preferably a chemically modified cyclodextrin, by substituting one or more hydroxy groups with alkyl, aryl, arylalkyl, glycoside groups or by etherification, esterification with alcohols or aliphatic acids. More preferred cyclodextrins can be selected among, but not only, Hydroxypropyl-β-cyclodextrin or HP-βCD (available from Roquette), Randomly Methylated-β-cyclodextrin or Rameb-CD (available from Cyclolab), sulfobutylether-β-cyclodextrin or Captisol (available from Cydex) or methyl-β-cyclodextrin.

The nanoparticles of the invention may additionally comprise further pharmaceutically acceptable excipients as those generally used in the field, such as surfactives, stabilizing agents or tensioactives such as dextran or poloxamer or other non ionic surfactive agents (like polysorbate, sorbitan esters or others). Poloxamers are preferred, such as Poloxamer 188 (also named pluronic F68).

The size of the nanoparticles generally depends on the concentration in the cyclic oligosaccharide capable of complexing the active principle, the pH of the polymerization medium, and the stirring rate. The size of the nanoparticles is lower than 1 micrometer, preferably comprised between 20 nm to 1000 nm and more preferably between 50 nm to 700 nm.

It is thus possible by conducting standard preliminary tests to adjust the size of the nanoparticles, depending of the particularly desired effect.

The nanoparticles of the present invention preferably comprise, in weight, percentage:
- from 0.1 to 30% of said camptothecin derivative;
- from 10 to 85% of said polymer;
- from 0.1 to 70% of said cyclic oligosaccharide capable of complexing said camptothecin derivative.
Additionally, the nanoparticles of the invention may also comprise:
- from 0 to 60% of excipients, such as poloxamer(s); and/or
- from 0 to 2% of acid(s), such as citric acid.

According to the present invention, the camptothecin derivatives refer to camptothecin analogs such as irinotecan, topotecan, hexatecan, silatecan, lutortecan, karenitecin (BNP1350), gimatecan (ST1481), belotecan (CKD602) or their pharmaceutically acceptable salts. Irinotecan and topotecan are preferred. Irinotecan is more preferred.

Cancer herein refers to any maligant proliferative cell disorders and include any kind of colorectal cancer, lung cancers, stomach cancer, pancreas cancer, non-Hodgkin lymphoma, uterine cervix cancer, head and neck cancers, brain cancer, breast cancer and ovary cancer.

As used herein, the term "patient" refers to either an animal, such as a valuable animal for breeding, company or preservation purposes, or preferably a human or a human child, which is afflicted with, or has the potential to be afflicted with one or more diseases and conditions described herein.

As used herein, a "therapeutically effective amount" refers to an amount of a compound of the present invention which is effective in preventing, reducing, eliminating, treating or controlling the symptoms of the herein-described diseases and conditions. The term "controlling" is intended to refer to all processes wherein there may be a slowing, interrupting, arresting, or stopping of the progression of the diseases and conditions described herein, but does not necessarily indicate a total elimination of all disease and condition symptoms, and is intended to include prophylactic treatment.

As used herein, the term "pharmaceutically acceptable" refers to those compounds, materials, excipients, compositions or dosage forms which are, within the scope of sound medical judgment, suitable for contact with the tissues of human beings and animals without excessive toxicity, irritation, allergic response or other problem complications commensurate with a reasonable benefit/risk ratio.

As used herein, "pharmaceutically acceptable salts" refer to derivatives of the disclosed compounds wherein the parent compound is modified by making acid or base salts thereof. The pharmaceutically acceptable salts include the conventional non-toxic salts or the quaternary ammonium salts of the parent compound formed, for example, from non-toxic inorganic or organic acids. For example, such conventional non-toxic salts include those derived from inorganic acids such as hydrochloric, hydrobromic, sulfuric, sulfamic, phosphoric, nitric and the like; and the salts prepared from organic acids such as acetic, propionic, succinic, tartaric, citric, methanesulfonic, benzenesulfonic, glucoronic, glutamic, benzoic, salicylic, toluenesulfonic, oxalic, fumaric, maleic, lactic and the like. Further addition salts include ammonium salts such as tromethamine, meglumine, epolamine, etc., metal salts such as sodium, potassium, calcium, zinc or magnesium.

The pharmaceutically acceptable salts of the present invention can be synthesized from the parent compound which contains a basic or acidic moiety by conventional chemical methods. Generally, such salts can be prepared by reacting the free acid or base forms of these compounds with a stoichiometric amount of the appropriate base or acid in water or in an organic solvent, or in a mixture of the two. Generally, non-aqueous media like ether, ethyl acetate, ethanol, isopropanol, or acetonitrile are preferred. Lists of suitable salts are found in Remington's Pharmaceutical Sciences, 17th ed., Mack Publishing Company, Easton, PA, 1985, p. 1418, the disclosure of which is hereby incorporated by reference.

As used herein, "pharmaceutically acceptable excipient" includes any carriers, diluents, adjuvants, or vehicles, such as preserving or antioxidant agents, fillers, disintegrating agents, wetting agents, emulsifying agents, suspending agents, solvents, dispersion media, coatings, antibacterial and antifungal agents, isotonic and absorption delaying agents and the like. The use of such media and agents for pharmaceutical active substances is well-known in the art. Except insofar as any conventional media or agent is incompatible with the active ingredient, its use in the therapeutic compositions is contemplated. Supplementary active ingredients can also be incorporated into the compositions as suitable therapeutic combinations.

The present invention also concerns the corresponding methods of treatment comprising the oral administration of a nanoparticle of the invention together with a pharmaceutically acceptable excipient to a patient in the need thereof.

The identification of those subjects who are in need of treatment of herein-described diseases and conditions is well within the ability and knowledge of one skilled in the art. A veterinarian or a physician skilled in the art can readily identify, by the use of clinical tests, physical examination, medical/family history or biological and diagnostic tests, those subjects who are in need of such treatment.

A therapeutically effective amount can be readily determined by the attending diagnostician, as one skilled in the art, by the use of conventional techniques and by observing results obtained under analogous circumstances. In determining the therapeutically effective amount, a number of factors are considered by the attending diagnostician, including, but not limited to: the species of subject; its size, age, and general health; the specific disease involved; the degree of involvement or the severity of the disease; the response of the individual subject; the particular compound administered; the mode of administration; the bioavailability characteristic of the preparation administered; the dose regimen selected; the use of concomitant medication; and other relevant circumstances.

The amount of the camptothecin derivatives, which is required to achieve the desired biological effect, will vary depending upon a number of factors, including the chemical characteristics (e.g. hydrophobicity) of the compounds employed, the potency of the compounds, the type of disease, the species to which the patient belongs, the diseased state of the patient, the route of administration, the bioavailability of the compound by the chosen route, all factors which dictate the required dose amounts, delivery and regimen to be administered.

In the context of the invention, the term "treating" or "treatment", as used herein, means reversing, alleviating, inhibiting the progress of, or preventing the disorder or condition to which such term applies, or one or more symptoms of such disorder or condition.

In general terms, the compounds of this invention may be provided in an aqueous solution or suspension containing 0.05 to 10 % w/v compound. Typical dose ranges are from 1 µg/kg to 0.1 g/kg of body weight per day; a preferred dose range is from 0.01 mg/kg to 10 mg/kg of body weight per day or an equivalent dose in a human child. The preferred dosage of drug to be administered is likely to depend on such variables as the type and extent of progression of the disease or disorder, the overall health status of the particular patient, the relative biological efficacy of the compound selected, the formulation of the compound, the pharmacokinetic properties of the compound by the chosen delivery route, schedule of administrations (number of repetitions in a given period of time), and concomitant treatment.

The compounds of the present invention are also capable of being administered in unit dose forms, wherein the term "unit dose" means a single dose which is capable of being administered to a patient, and which can be readily handled and packaged, remaining as a physically and chemically stable unit dose comprising either the active compound itself, or as a pharmaceutically acceptable composition, as described hereinafter. As such, typical total daily dose ranges are from 0.01 to 100 mg/kg of body weight. By way of general guidance, unit doses for humans range from 1 mg to 3000 mg per day. Preferably, the unit dose range is from 1 to 1000 mg administered one to six times a day, and even more preferably from 10 mg to 1000 mg, once a day. Compounds provided herein can be formulated into pharmaceutical compositions by admixture with one or more pharmaceutically acceptable excipients. Such unit dose compositions may be prepared for use by oral administration, particularly in the form of tablets, capsules, oral suspension, powder to resuspend or syrup.

The compositions may conveniently be administered in unit dosage form and may be prepared by any of the methods well-known in the pharmaceutical art, for example, as described in Remington: The Science and Practice of Pharmacy, 20th ed.; Gennaro, A. R., Ed.; Lippincott Williams & Wilkins: Philadelphia, PA, 2000.

For oral administration, tablets, pills, powders, capsules, suspension, syrup and the like can contain one or more of any of the following vehicles, or compounds of a similar nature: a binder such as microcrystalline cellulose, or gum tragacanth; a diluent such as starch or lactose; a disintegrant such as starch and cellulose derivatives; a lubricant such as magnesium stearate; a glidant such as colloidal silicon dioxide; a sweetening agent such as sucrose or saccharin; or a flavoring agent. Capsules can be in the form of a hard capsule or soft capsule, which are generally made from gelatin blends optionally blended with plasticizers, as well as a starch capsule. In addition, dosage unit forms can contain various other materials that modify the physical form of the dosage unit, for example, coatings of sugar, shellac, or enteric agents. Other oral dosage forms syrup or elixir may contain sweetening agents, preservatives, dyes, colorings, and flavorings.

According to a still further object, the present invention also concerns a medicament comprising at least one nanoparticle according to the invention in a pharmaceutically acceptable vehicle, said medicament being for oral administration.

According to a still further object, the present invention also concerns a medicament comprising at least one nanoparticle according to the invention in a pharmaceutically acceptable vehicle for the treatment and/or the prevention of cancer, said treatment and/or prevention comprising the oral administration of said medicament.

According to a further object of the invention, the treatment may also include the administration of one or more further anticancer agent, such as, but not limited to, 5-fluorouracil or other antimetabolite fluoropyrimidine like capecitabine or Tegafur-Uracile ("UFT").

According to a further object, the present invention also concerns the process of preparation of the nanoparticles of the invention, said process comprising the steps consisting in:
- preparing a polymerisation medium comprising at least one acid, Cyclodextrins and Poloxamer 188;
- mixing said active ingredient with the polymerisation medium;
- mixing with the monomer of said polymer;
- allowing polymerisation to occur.

### DESCRIPTION OF THE DRAWINGS

**Figure 1** illustrates the tumor evolution of treated mice where Relative Tumor Volume (RTVm) represents the tumor evolution related to the volume registered at the beginning of the treatment with a formulation of the invention.
**Figure 2** illustrates the orthotopic tumor volume evolution of treated mice assessed at two sacrifice time, when administered with a formulation of the invention.
**Figure 3** illustrates the weight evolution of treated mice where Relative Body Weight (RBWm) represents the body weight evolution related to the weight registered at the beginning of the experimentation per group, when administered with a formulation of the invention.
**Figure 4** illustrates the tumor evolution of treated mice where Relative Tumor Volume (RTVm) represents the tumor evolution related to the volume registered at the beginning of the treatment with a formulation of the invention.

### EXAMPLES

The following examples are given as a non limiting illustration of the present invention.

### Example 1: Raw Materials, and Main Protocols

### Raw Materials

| | **Provider** | **Sample number** |
|---|---|---|
| Irinotecan | Interchemical / Haorui | IRN 01-001 |
| HP-β Cyclodextrin | Roquette | ND |
| Rameb Cyclodextrin | Cyclolab | CYL 22-47 |
| Monohydrated citric acid | Cooper | P51825/1 |
| Poloxamer 188 | ND | POL01-003 |
| Monorex® | BioAlliance Pharma | YGZ 004-02 |

### Method for preparing the polymerization Medium (pH comprises between 2 and 3.5)

- Preparation of citric acid 0.1 M
- Addition of poloxamer 188 (concentration between 0,5 and 1%) in the previous solution, under stirring and till complete dissolution occurs
- Addition of Cyclodextrin (concentration between 0.1 and 1 %)
- Adjustment to the required pH
- Filtration on 0.2 µm filters.

### Method for preparing Irinotecan-SRN (5 ml batches)

- In a 10 ml flask, add 5 ml of Irinotecan between 0.5 and 2 mg/ml in the polymerization medium
- Magnetic stirring
- Add slowly 50 to 200 µl of Monorex® under stirring
- Let polymerization occurs up to 24h period of time, under magnetic stirring and room temperature
- Filtration onto 2 µm filters

### Example 2: encapsulations studies and optimization: Incidence of increasing quantities of IRN

**Protocol:** Solutions of 0.5, 0.75, 1, 2 & 5 mg/ml of IRN in pH 3.5 polymerization medium were prepared and placed in 10 ml flasks (5 ml of solution per flask). Polymerization were launched by adding dropwise 50 µl of Monorex® under magnetic stirring. After 24 h, suspensions were collected and analyzed by HPLC before and after filtration on 2 µm filters, before and after centrifugation at 50.000 rpm, 30 min at 20°C.

**Results**: Increasing quantities of irinotecan in a fixed quantity of monomer, lead to decreasing values of encapsulation yields (data not shown). Best results are obtained with 0,5 to 1,5 mg/ml IRN solutions with 50 µl of Monorex® with encapsulation yields >95%.

### Example 3: Preliminary in vitro & in vivo results

### 3.1. In vitro cytotoxicity results of IRN-SRN on HT29 cells

The effect of the irinotecan-SRN was tested. SRN alone, irinotecan-SRN (with the weight ratio irinotecan/polymer equal to 1/20) as well as irinotecan alone were put in contact with growing HT29 cells for 4 days and the IC₅₀ were determined. Three different runs were performed and IC₅₀ (inhibition concentration 50%) was estimated.

The different efficacies determined are listed below :

**Table 1: In vitro antitumor activity of the three test articles (IC50 values)**

| | **Test article** | **Inhib. Conc.** | **Experiment number** | | | | | | **Mean IC 50** | |
|---|---|---|---|---|---|---|---|---|---|---|
| | | | **GF411** | | **GF464** | | **GF522** | | | |
| | | | [IRN] mg/mL | [Polymer] mg/mL | [IRN] mg/mL | [Polymer] mg/mL | [IRN] mg/mL | [Polymer] mg/mL | [IRN] mg/mL | [Polymer] mg/mL |
| 1 | IRN-SRN | IC50 | 0.80 | 15,99 | 0,83 | 16,66 | 1,14 | 22,75 | 0,82 | 18,47 |
| 2 | IRN alone | IC50 | 0.75 | | 0,80 | | 0,85 | | 0,80 | |
| 3 | SRN alone | IC50 | | 33,86 | | 36,57 | | 34,94 | | 35,1 |

In vitro, irinotecan-SRN exhibited a similar anticancer activity as irinotecan alone in the human colon cancer cell line HT-29, suggesting that entrapment of Irinotecan into the nanoparticles did not reduce its activity. One should however keep in mind that SN38 is the active metabolite of IRN and in order to be active it has to be metabolized by carboxyl esterase enzymes. So this could be a limitation of the *in vitro* tests on cells.

It should be notice that blank nanoparticles (SRN alone) displayed a cytotoxic activity *in vitro.* Indeed, it has been described that the degradation of blank nanoparticles leads to polycyanoacrylate acid, which is known to be cytotoxic *in vitro.*

This *in vitro* study on the human colon cancer cell line HT-29 confirms the fact that irinotecan encapsulated in nanoparticles kept its efficacy and was not modified by the polymerisation process.

### 3.2. In vivo studies of IRN-SRN on xenografted mice

### 3.2.1. Single Maximum Tolerated Dose (MTD) studies:

### Oral administration of IRN-SRN

Single dose MTD evaluation studies were first conducted. Three dose escalations were orally tested, 50 mg/kg; 100 mg/kg and 200 mg/kg. Each dose was tested on a group of 3 animals and the animals were monitored for 2 weeks, clinical toxicity signs were observed and the animals weight taken every two days.

At 50 mg/kg and 100 mg/kg orally, no signs of toxicity appeared and no body weight loss was observed. At 200 mg/kg, after 4 days two of the animals are at 94% and one is at 91.5% of their initial body weight but there were no apparent clinical toxicity signs. A reference group was dosed orally with CAMPTO® (IRN in solution) and showed no weight loss or clinical toxicity signs.

The Maximum Tolerated dose for oral dosage of IRN-SRN was 200 mg/kg in this experimental model.

### 3.2.2. MTTD (Maximum Total Tolerated Doses) Studies for oral administration of IRN-SRN

The schedule of the MTTD studies was one administration per week during three weeks. Each dose was tested on a group of 3 animals and the animals were monitored for 2 weeks after the end of the last treatment, clinical toxicity signs were observed and the animals weight taken every two days.

MTTD studies planned had the following schedule:

**Table 2: Schedule of MTTD studies and clinical manifestations**

| | **Treatment** | | **Dose level [mg/kg BW]** | **Schedule** | **Clinical symptoms** |
|---|---|---|---|---|---|
| 1 | Vehicle | iv | 10 | J=0, 7, 14 | No |
| 2 | IRN-SRN | po | 200 | J=0, 7, 14 | No clinical sign |
| 3 | IRN | iv | 25 | J=0, 7, 14 | No clinical sign |
| 4 | IRN | po | 200 | J=0, 7, 14 | No clinical sign |

For oral administration, the MTTD could not be determined due to the highest volume of dosing required. Therefore, a maximum feasible dose has been determined at 200 mg/kg in this experimental model.

### 3.3. Oral MTTD studies on xenografted mice

A tolerance and efficacy study on xenografted mice, in order to mimic as close as possible the *in vivo* model of colonic tumor, was carried out. As xenografted mice are very sensitive, the MTTD and efficacy study was done on mice with a subcutaneously implanted HT-29 colorectal human tumor cells.

The schedule was the following:

**Table 3: Schedule of MTTD studies and clinical manifestations**

| | **Treatment** | | **Dose level [mg/kg BW]** | **Schedule** | **Clinical symptoms** |
|---|---|---|---|---|---|
| 1 | Vehicle | po | 10 ml/kg BW | J=1 to 5 | No |
| 2 | IRN-SRN | po | 200 | J=1 to 5 | No clinical signs |
| 3 | IRN-SRN | po | 100 | J=1 to 5 | No clinical signs |
| 4 | IRN-SRN | po | 50 | J=1 to 5 | No clinical signs |
| 5 | IRN | po | 200 | J=1 to 5 | Important weight loss (>10%), treatment was interrupted |
| 6 | IRN | po | 100 | J=1 to 5 | Weight loss, but mice recovered the week after |

IRN-SRN administered orally every day for 5 days showed no clinical signs of toxicity (table 3). On the other hand, IRN induced an important weight loss.

As for the previous experiment with an administration schedule of three oral administration every week for 3 weeks, the MTTD could not be determined due to the highest volume of dosing required. Therefore, a maximum feasible dose has been determined at 200 mg/kg in this experimental model.

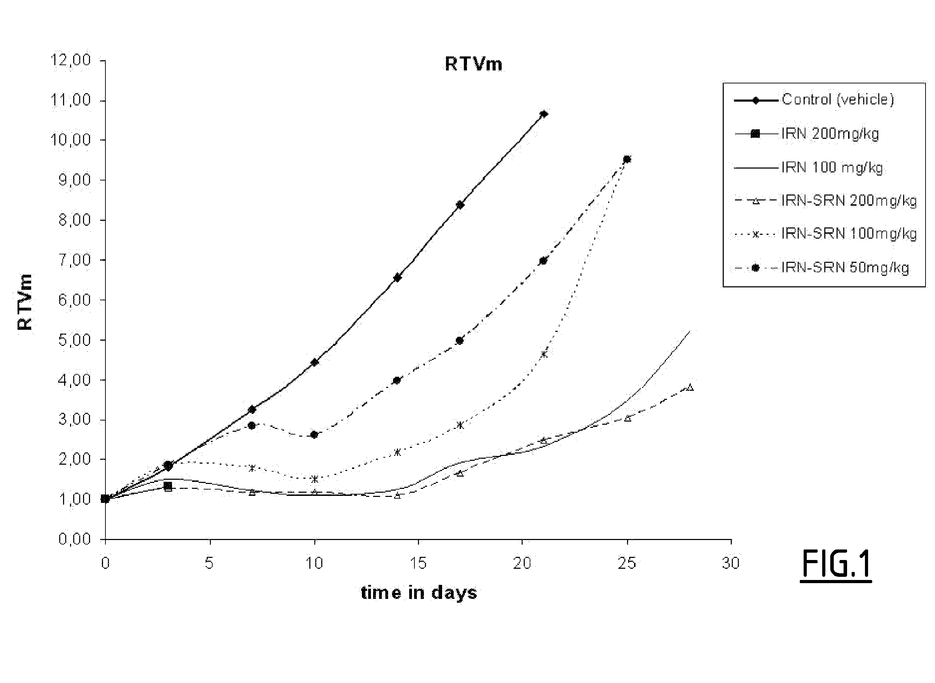

An examination of the tumor evolution was also considered during this experiment. Treatment with IRN-SRN (50, 100, and 200 mg/kg) induces a significant reduction of the tumor volumes (see figure 1).

At this step, IRN-SRN have shown interesting properties. Indeed, they not only have a dose dependent efficacy and an antitumor effect comparable to CAMPTO®, but they are also better tolerated.

### 3.4. Efficacy study of IRN-SRN against HT29 tumor xenografts growing orthotopically

The objective is to test the efficacy of IRN-SRN administered per os on HT-29 colorectal tumors growing orthotopically. Dosage and schedule used were chosen from results of tolerance study.

Orthotopic grafts: tumor fragments about 30 mm³ are sutured against the caecum wall as described above. Groups of 15 mice are randomly affected to the treatments 2 weeks post-grafting.

The treatment were adjusted to lower doses if the animals loose weight (>10% for 72 consecutive hours). In each group, a pool of 7-8 mice were sacrificed at day 19 and day 45 after start of treatment. Tumor samples were fixed in 10% formol and processed for histological examination.

| **Group** | **Treatment** | **Dosage, schedule** | **Nb of mice** |
|---|---|---|---|
| 1 | Control | vehicle, po, qdx5 | 15 |
| 2 | IRN | 100 mg/kg, po, qdx5 | 15 |
| 5 | IRN-SRN | 200 mg/kg, po, qdx5 | 15 |

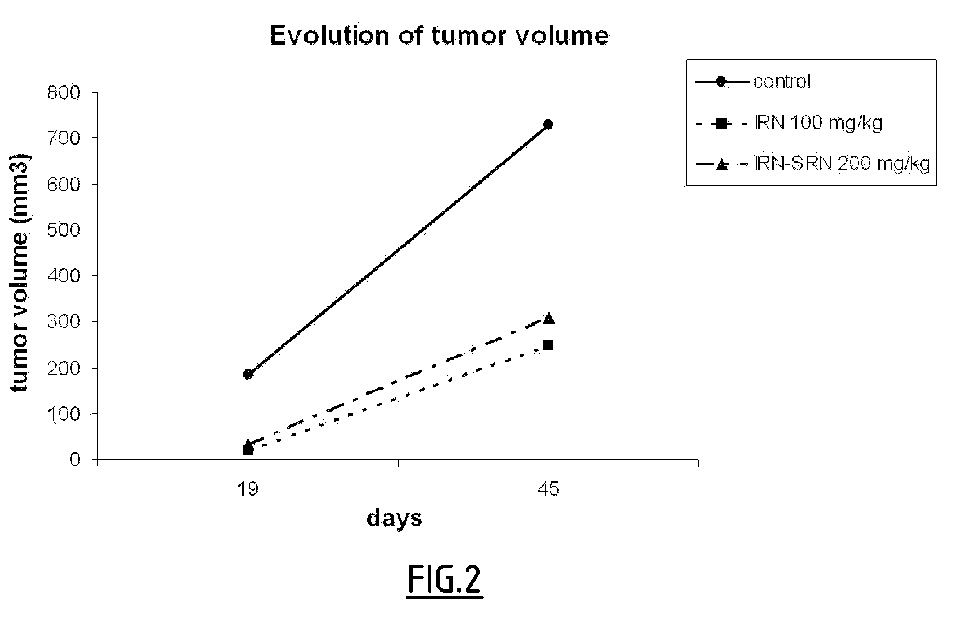

Treatment with IRN-SRN (200 mg/kg) induces a significant reduction of the tumor volumes (see figure 2).

### Example 4: In vivo Anti-tumor effect of IRN-SRN and Irinotecan in the HT29 xenograft model of human colorectal adenocarcinoma

### 4.1. Material & Methods

### Identification of animals and formulation of dosage groups

All animals are weighed before each experiment and are subdivided into the different dosage groups.

Each cage is identified by a paper tag indicating: experiment code, cage number, mice number, tumor code, name of the test item, dose and route of administration.

### Tumor induction

The HT29 colorectal tumor cell line has been established as a subcutaneously growing tumor xenograft into nude mice.

Tumor xenografts are maintained by serial transplantation into immunodeficient mice. Mice received subcutaneous grafts of tumor fragments originated from a previous passage. Fragments for this assay will originate from 5 donor mice bearing the previous tumor passage and sacrificed when the tumor reached 12 to 15 mm of diameter. All mice from the same experiment were implanted on the same day. It was planned to include at least 10 mice per group.

Tumor-bearing donor mice were sacrificed by cervical dislocation. The tumor was aseptically excised. Tumors were deposited in a Petri dish containing culture medium and dissected carefully to remove the fibrous capsule usually surrounding the tumor. Necrotic tumors were rejected. Tumor tissue was maintained in culture medium during the transplantation procedure. One tumor led up to 8 transplants, each fragment measuring approximately 40 mm³.

Subcutaneous implantations were performed aseptically. After anaesthesia with ketamine/xylazine, and sterilisation of the skin with a betadine solution, the skin was incised at the level of the interscapular region, and a fragment of tumor was placed in the subcutaneous tissue. Skin was closed with clips.

### Inclusion criteriae and randomization

Only healthy mice aged 7 to 9 weeks and weighing at least 20 g were included in the study. At the step of transplantation, tumor fragments were randomly distributed onto nude mice and were be individually identified by a number and allocated to a tumor fragment. Treatments were randomly attributed to boxes housing 3 to 5 mice.

### Chemotherapy protocol

### • Drug administration

IRN-SRN and IRN were administered by oral or intravenous route following the indicated regimens. Mice received variant volumes of IRN-SRN in order to obtain the wanted doses (about 125 to 750 µL). Irinotecan was diluted in order to administrate 500 µL by oral route.

### • Vehicle

Control animals received the vehicle used to prepare the IRN-SRN solution (polymerization medium).

### Experimental protocols

### • Tolerance study (Step 1)

The objectives were to test the tolerance to Irinotecan and IRN-SRN given either iv or per os to nude mice bearing HT29 tumors, according to the defined dosage and administration route.

When subcutaneous HT29 xenografts are detectable and reach a mean tumor volume of 150 mm3, groups of mice are randomly affected to the treatments. Mice without tumors were eliminated.

The treatment were adjusted to lower doses if the animals loose weight (>15% for 72 consecutive hours).

Mice in the control group received vehicle under a 750 µl volume.

| **Group** | **Treatment** | **Dosage, schedule** | **Nb of mice** |
|---|---|---|---|
| 1 | Control Vmax | Vehicle, po, qdx5 | 5 |
| 2 | IRN | 20 mg/kg, iv, (qwk)x2 | 5 |
| 3 | IRN | 100 mg/kg, po, qdx5 | 5 |
| 4 | IRN-SRN | 50 mg/kg, po, qdx5 | 5 |
| 5 | IRN-SRN | 100 mg/kg, po, qdx5 | 5 |
| 6 | IRN-SRN | 200 mg/kg, po, qdx5 | 5 |
| 7 | IRN-SRN | 300 mg/kg, po, qdx5 | 5 |

### • Efficacy study of IRN-SRN against HT29 tumor xenografts growing subcutaneously (Step 2)

The objectives were to test the efficacy of IRN-SRN administered per os on HT-29 colorectal tumors growing subcutaneously. Dosage and schedule used were chosen from results of tolerance study.

Groups of 10 mice were randomly affected to the treatment groups to give identical mean tumor volumes between groups when tumor volume is between 60-150 mm³. Treatments started on the next day following group affectation (Day 1).

The treatment will be adjusted to lower doses if the animals loose weight (>15% for 72 consecutive hours).

| **Group** | **Treatment** | **Dosage, schedule** | **Nb of mice** |
|---|---|---|---|
| 1 | Control | vehicle, po, qdx5 | 10 |
| 2 | IRN | 100 mg/kg, po, qdx5 | 10 |
| 3 | IRN | 20 mg/kg, iv, (qwk)x2 | 10 |
| 4 | IRN-SRN | 300 mg/kg, po, qdx5 | 10 |
| 5 | IRN-SRN | 100 mg/kg, po, qdx5 | 10 |
| 6 | IRN-SRN | 33 mg/kg, po, qdx5 | 10 |
| 7 | SRN-Control po | qdx5 | 6 |

### Bio-distribution study of IRN-SRN (Step 3)

Blood was sampled by cardiac puncture on xylamine-ketamine-anesthesized mice 5min, 10min, 15min, 30min, 60min, 2h, 4h, 8h, 16h, 30h, and 48h after a single iv injection, or 15min, 30min, 60min, 2h, 4h, 8h, 16h, 30h, and 48h after a single po administration.

Tumors were dissected and flash-frozen 60min, 4h, 8h, 16h, 30h, and 48h after a single iv injection or po administration.

Three mice were used per time-point. Only one sampling will be performed on the control group.

| **Group** | **Treatment** | **Dosage, schedule** | **Nb of mice** |
|---|---|---|---|
| 1 | Control | vehicle, po | 3 |
| 2 | IRN | 100 mg/kg, po | 27 |
| 3 | IRN | 20 mg/kg, iv | 33 |
| 4 | IRN-SRN | 300 mg/kg, po | 27 |
| 5 | IRN-SRN | 100 mg/kg po | 27 |

Plasma samples were prepared for CPT11 and its active metabolite SN38 assay.

### Tumor growth

To evaluate the antitumor activity of drugs on human xenografts, tumor volumes are evaluated by measuring biweekly tumor diameters with a calliper. The formula TV (mm³) = [length (mm) x width (mm)2]/2 is used, where the length and the width were the longest and the shortest diameters of each tumor, respectively.

Relative tumor volume (RTV): is calculated as the ratio of the volume at the time t divided by the initial volume at day 1 and multiplied by 100. Curves of mean RTV as a function of time in treated and control groups are generated and presented in the report.

### Toxicity parameters

Toxicity of the different treatments are determined as: body weight loss percent (% BWL max) = 100 - (mean BWx/mean BW1x 100), where BWx is the mean BW at the day of maximal loss during the treatment and BW1 is the mean BW on the 1 st day of treatment.

Lethal toxicity is any death in treated group.

### Clinical observations

### • Mortality

Animals are inspected every day for mortality.

### • Clinical signs

Mice are observed daily for physical appearance, behaviour and clinical changes.

Clinical observations are made in order to detect abnormalities related to the involvement of tegumental, digestive, musculoskeletal, respiratory, genitourinary apparatus and central nervous system.

All signs of illness, together with any behavioural change or reaction to treatment, are recorded for each animal. All clinical signs are recorded for individual animal, in the laboratory notebook throughout the whole study.

### • Body weight

All animals are weighted during the whole treatment period, in order to adjust the volume of drug administration and to calculate the percent body weight loss due to the different treatments.

### 4.2. Results

| | Control p.o. | | IRN 100mg/kg p.o | | IRN 20 mg/kg i.v. | |
|---|---|---|---|---|---|---|
| Day | RBW m | SD | RBW m | SD | RBW m | SD |
| 0 | 1,00 | 0,00 | 1,00 | 0,00 | 1,00 | 0,00 |
| 1 | 1,01 | 0,01 | 0,98 | 0,01 | 1,00 | 0,01 |
| 2 | 0,99 | 0,01 | 0,93 | 0,01 | 0,95 | 0,01 |
| 3 | 1,03 | 0,01 | 0,90 | 0,01 | 0,95 | 0,01 |
| 4 | 1,01 | 0,01 | 0,88 | 0,01 | 0,96 | 0,01 |
| 7 | 1,00 | 0,01 | 0,83 | 0,03 | 0,96 | 0,02 |
| 10 | 0,97 | 0,02 | 0,90 | 0,03 | 0,95 | 0,02 |
| 15 | 0,99 | 0,01 | 1,00 | 0,02 | 0,97 | 0,02 |

| | Control p.o. | | IRN-SRN 50mg/kg p.o. | | IRN-SRN 100mg/kg p.o. | |
|---|---|---|---|---|---|---|
| Day | RBW m | SD | RBW m | SD | RBW m | SD |
| 0 | 1,00 | 0,00 | 1 | 0 | 1 | 0 |
| 1 | 1,01 | 0,01 | 1,01 | 0,01 | 1,00 | 0,00 |
| 2 | 0,99 | 0,01 | 0,98 | 0,01 | 0,97 | 0,01 |
| 3 | 1,03 | 0,01 | 0,97 | 0,02 | 0,98 | 0,01 |
| 4 | 1,01 | 0,01 | 1,00 | 0,01 | 0,97 | 0,01 |
| 7 | 1,00 | 0,01 | 0,98 | 0,01 | 0,97 | 0,01 |
| 10 | 0,97 | 0,02 | 0,97 | 0,02 | 0,96 | 0,01 |
| 15 | 0,99 | 0,01 | 0,98 | 0,04 | 1,03 | 0,02 |

**Table 4: Relative body weight (RBW) as a function of time**

| | Control p.o. | | IRN-SRN 200mg/kg p.o. | | IRN-SRN 300mg/kg p.o. | |
|---|---|---|---|---|---|---|
| Day | RBW m | SD | RBW m | SD | RBW m | SD |
| 0 | 1,00 | 0,00 | 1 | 0 | 1 | 0 |
| 1 | 1,01 | 0,01 | 0,98 | 0,004 | 0,984 | 0,007 |
| 2 | 0,99 | 0,01 | 0,96 | 0,005 | 0,948 | 0,006 |
| 3 | 1,03 | 0,01 | 0,95 | 0,012 | 0,968 | 0,009 |
| 4 | 1,01 | 0,01 | 0,95 | 0,014 | 0,984 | 0,012 |
| 7 | 1,00 | 0,01 | 0,96 | 0,008 | 0,9723 | 0,018 |
| 10 | 0,97 | 0,02 | 0,97 | 0,0156 | 0,961 | 0,026 |
| 15 | 0,99 | 0,01 | 1,04 | 0,0128 | 1,038 | 0,017 |

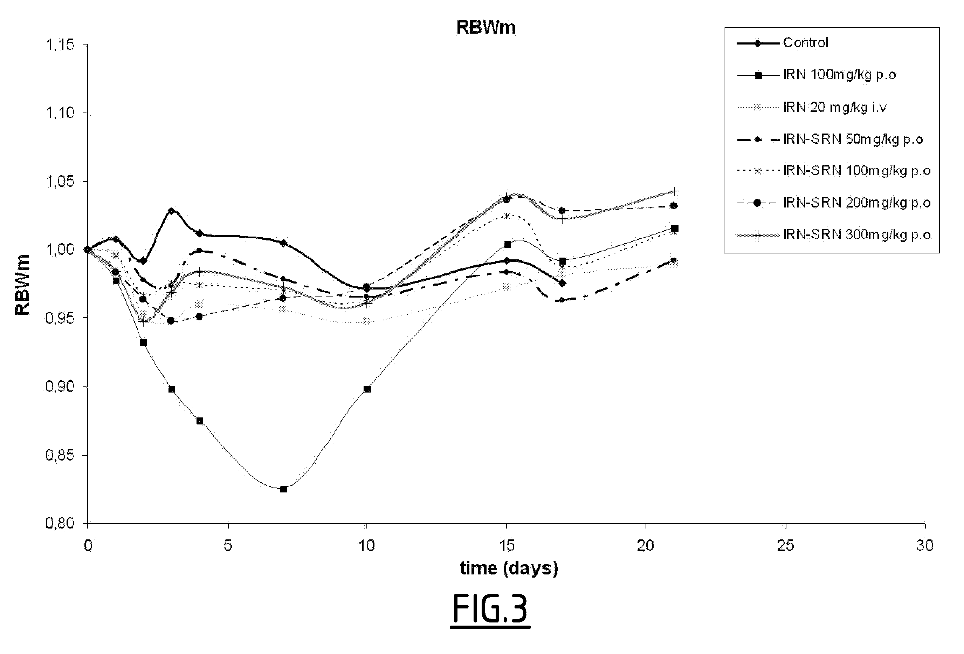

No body weight loss was observed in the IRN-SRN groups (50, 100, 200 and 300 mg/kg) after oral administration whereas more than 15% body weight loss was observed in the oral IRN alone group (see figure 3). It indicated that IRN-SRN is better tolerated than IRN alone after oral administration. It confirms that IRN-SRN could be able to reduce side effects of oral administration of irinotecan.

| | Control p.o. | | IRN 100mg/kg p.o. | | IRN 20 mg/kg i.v | |
|---|---|---|---|---|---|---|
| Day | RTV m | SD | RTV m | SD | RTV m | SD |
| 0 | 1 | 0 | 1 | 0 | 1 | 0 |
| 3 | 3,16 | 0,68 | 2,20 | 0,36 | 2,02 | 0,26 |
| 7 | 4,90 | 0,72 | 1,74 | 0,26 | 2,26 | 0,43 |
| 10 | 7,01 | 1,31 | 1,30 | 0,13 | 2,90 | 0,60 |
| 15 | 12,35 | 2,31 | 1,98 | 0,22 | 4,71 | 1,07 |

| | Control p.o. | | IRN-SRN 50mg/kg p.o. | | IRN-SRN 100mg/kg p.o. | |
|---|---|---|---|---|---|---|
| Day | RTV m | SD | RTV m | SD | RTV m | SD |
| 0 | 1 | 0 | 1 | 0 | 1 | 0 |
| 3 | 3,16 | 0,68 | 2,48 | 0,33 | 2,59 | 0,38 |
| 7 | 4,90 | 0,72 | 2,59 | 0,47 | 2,70 | 0,24 |
| 10 | 7,01 | 1,31 | 3,89 | 0,89 | 2,17 | 0,29 |
| 15 | 12,35 | 2,31 | 7,69 | 2,88 | 4,13 | 0,62 |

**Table 5: Relative tumor volume (RTV) as a function of time**

| | Control p.o | | IRN-SRN 200mg/kg p.o. | | IRN-SRN 300mg/kg p.o. | |
|---|---|---|---|---|---|---|
| Day | RTV m | SD | RTV m | SD | RTV m | SD |
| 0 | 1 | 0 | 11 | 0 | 1 | 0 |
| 3 | 3,16 | 0,68 | 2,40 | 0,60 | 1,67 | 0,19 |
| 7 | 4,90 | 0,72 | 1,70 | 0,31 | 1,80 | 0,21 |
| 10 | 7,01 | 1,31 | 1,30 | 0,26 | 1,31 | 0,15 |
| 15 | 12,35 | 2,31 | 2,25 | 0,42 | 1,57 | 0,44 |

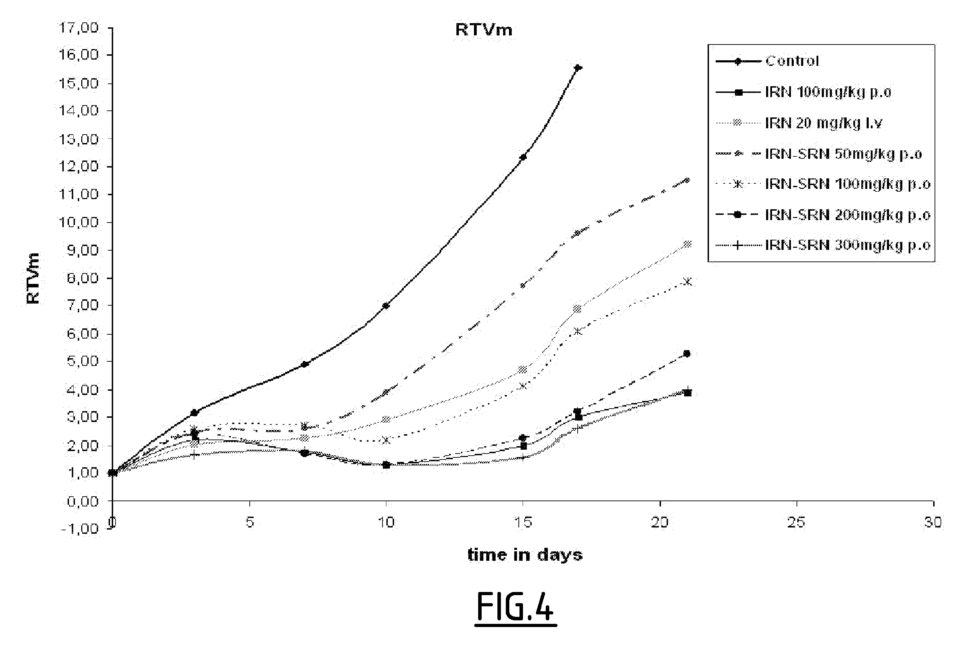

Treatment with IRN-SRN (50, 100, 200 and 300 mg/kg) also induces a significant reduction of the tumor volumes (see figure 4). Moreover administration of different doses show a dose related effect of IRN-SRN. Indeed efficacy of the treatment increases with the administrated dose of IRN-SRN. Oral IRN-SRN at 200 and 300 mg/kg is as efficient as oral IRN alone at 100 mg/kg and is better tolerated.

## Claims

1. Nanoparticles comprising at least one camptothecin derivative as an active ingredient, at least one polymer and at least one cyclic oligosaccharide capable of complexing said camptothecin derivative, said nanoparticles being for therapeutic oral administration.

2. Nanoparticles comprising at least one camptothecin derivative as an active ingredient, at least one polymer and at least one cyclic oligosaccharide capable of complexing said camptothecin derivative for the treatment and/or the prevention of cancer, said treatment and/or prevention comprising the oral administration of said nanoparticles.

3. Nanoparticles according to claim 1 or 2, wherein said polymer is chosen from the poly(alkylcyanoacrylate) group in which the alkyl group, linear or branched, comprises 1 to 12 twelve carbon atoms.

4. Nanoparticles according to claim 1, 2 or 3, wherein said polymer is a poly(isohexylcyanoacrylate).

5. Nanoparticles according to any one of the preceding claims, wherein said cyclic oligosaccharide capable of complexing the active ingredient is a cyclodextri n.

6. Nanoparticles according to anyone of the preceding claims, wherein said camptothecin derivative is irinotecan or topotecan, or their pharmaceutically acceptable salt.

7. Nanoparticles according to anyone of the preceding claims, wherein said camptothecin derivative is irinotecan or its pharmaceutically acceptable salt.

8. Nanoparticles according to anyone of the preceding claims wherein they further comprise at least one pharmaceutically acceptable stabilizing agent, chosen from tensioactive or surfactive agent.

9. Nanoparticles according to claim 8, wherein said tensioactive or surfactive agent is a poloxamer.

10. Nanoparticles according to anyone of the preceding claims comprising :
- irinotecan;
- poly(isohexylcyanoacrylate);
- Poloxamer 188; and
- HP-βCD.

11. Nanoparticles according to anyone of the preceding claims, wherein said cancer includes colorectal cancer.

12. Nanoparticles according to anyone of the preceding claims, wherein said treatment includes the administration of one or more further anticancer agent.

13. Nanoparticles according to anyone of the preceding claims, wherein said further anticancer agent is 5-fluorouracil.

14. Medicament comprising at least one nanoparticle according to anyone of the preceding claims in a pharmaceutically acceptable vehicle, said medicament being for oral administration.

15. Medicament comprising at least one nanoparticle according to anyone of the preceding claims in a pharmaceutically acceptable vehicle for the treatment and/or the prevention of cancer, said treatment and/or prevention comprising the oral administration of said medicament.
